# EUROPEAN PATENT APPLICATION

(11) **EP 4 670 692 A1**
(43) Date of publication of application: **31.12.2025**
(21) Application number: 24780565.8
(22) Date of filing: 27.03.2024
(51) Int. Cl.: A61F 13/15, A61F 13/534, A61F 13/535

(54) **METHOD AND APPARATUS FOR MANUFACTURING ABSORBENT SHEET USED FOR ABSORBENT ARTICLE**

(30) Priority: 30.03.2023 JP 2023055786
(71) Applicant: Zuiko Corporation, Ibaraki-shi Osaka 5670082 (JP)
(72) Inventor: IWAMURA, Yosuke, Ibaraki-shi, Osaka 567-0082 (JP)
(74) Representative: SSM Sandmair
(86) International application number: PCT/JP2024/012498
(87) International publication number: WO 2024/204464

(57) **Abstract**

Provided are a method and an apparatus for manufacturing an absorbent sheet used for an absorbent article, with which it is possible to reduce the manufacturing cost of an absorbent sheet in which absorbent materials are arranged in two layers.

A first continuous sheet (2) is wound around a first conveyance roll (12a) and conveyed, a first absorbent material (3) is supplied to the first continuous sheet (2) being conveyed, and a second continuous sheet (4) is overlapped onto the first continuous sheet (2) so as to cover the first absorbent material (3) to form a first composite sheet (7). The first composite sheet (7) is wound around a second conveyance roll (14a) and conveyed, a second absorbent material (5) is supplied to the first composite sheet (7) being conveyed, and a third continuous sheet (6) is overlapped onto the first composite sheet (7) so as to cover the second absorbent material (5) to form a second composite sheet (8). The second composite sheet (8) is compressed in the thickness direction to form an absorbent sheet (9).

## Description

### TECHNICAL FIELD

The present invention relates to a method and an apparatus for manufacturing an absorbent sheet used for an absorbent article, and more particularly to a technique for manufacturing an absorbent sheet in which absorbent materials are arranged in two layers.

### BACKGROUND ART

Absorbent articles such as paper diapers are provided with absorbent bodies for absorbing and retaining liquids. In absorbent bodies, for example, powdered or granular absorbent materials such as SAP (Super Absorbent Polymer: highly absorbent macromolecular resin) are disposed between sheets made of nonwoven fabric or the like.

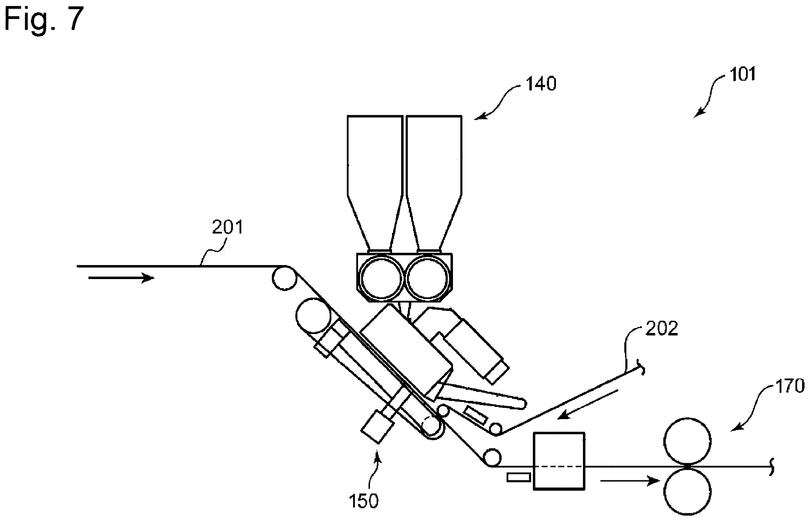

Such an absorbent body is formed by cutting an absorbent sheet. For example, using an apparatus 101 for manufacturing an absorbent sheet shown in Fig. 7, a first continuous sheet 201 is conveyed by a conveyor 150, SAP is supplied from an SAP supplying apparatus 140 onto the first continuous sheet 201, a second continuous sheet 202 is then overlapped onto and joined to the first continuous sheet 201 to which SAP has been supplied, and the joined first and second continuous sheets 201, 202 are then compressed using a compression apparatus 170 to form an absorbent sheet. The SAP supplying apparatus 140 is configured so that an opening for supplying SAP opens and closes at appropriate times (see, for example, Patent Literature 1).

### CITATION LIST

### PATENT LITERATURE

[Patent Literature 1] International Publication No. 2017/131025

### SUMMARY OF THE INVENTION

### PROBLEM TO BE SOLVED BY THE INVENTION

The variety of absorbent bodies can be increased by appropriately combining the type, amount, and arrangement pattern of the absorbent material placed in the absorbent body. Further variations can be achieved by placing the absorbent material in two layers and changing the combination of the type, amount, and arrangement pattern of the absorbent material in each layer.

Therefore, it is conceivable to prepare two apparatuses like the one shown in Fig. 7, and use each apparatus to form a composite sheet in which absorbent materials are arranged between two continuous sheets, and then overlap the two composite sheets to form an absorbent sheet, which can then be cut to form an absorbent body in which absorbent materials are arranged in two layers.

However, when manufactured in this manner, an absorbent sheet in which absorbent materials are arranged in two layers is formed using four continuous sheets, which increases the manufacturing cost.

In view of this situation, the problem that the present invention aims to solve is to provide a method and an apparatus for manufacturing an absorbent sheet used for an absorbent article, which can reduce the manufacturing cost of an absorbent sheet in which absorbent materials are arranged in two layers.

### MEANS FOR SOLVING THE PROBLEM

In order to solve the above problems, the present invention provides a method for manufacturing an absorbent sheet used for an absorbent article, configured as follows.

A method for manufacturing an absorbent sheet used for an absorbent article includes: (i) a first step of winding a first continuous sheet around a rotating first conveyance roll and conveying the first continuous sheet; (ii) a second step of supplying a first absorbent material to the first continuous sheet being wound around and conveyed by the first conveyance roll; (iii) a third step of overlapping a second continuous sheet onto the first continuous sheet so as to cover the first absorbent material supplied to the first continuous sheet, thereby forming a first composite sheet in which the first absorbent material is sandwiched between the first continuous sheet and the second continuous sheet; (iv) a fourth step of winding the first composite sheet around a rotating second conveyance roll and conveying the first composite sheet; (v) a fifth step of supplying a second absorbent material to the first composite sheet being wound around and conveyed by the first conveyance roll; (vi) a sixth step of overlapping a third continuous sheet onto the first composite sheet so as to cover the second absorbent material supplied to the first composite sheet, thereby forming a second composite sheet in which the first absorbent material is sandwiched between the first continuous sheet and the second continuous sheet, and the second absorbent material is sandwiched between the first continuous sheet and the third continuous sheet or between the second continuous sheet and the third continuous sheet; and (vii) a seventh step of compressing the second composite sheet in the thickness direction.

According to the above method, an absorbent sheet in which absorbent materials are arranged in two layers can be manufactured using three continuous sheets, which reduces the number of continuous sheets by one compared to when an absorbent sheet in which absorbent materials are arranged in two layers is manufactured using four continuous sheets, thereby reducing the manufacturing cost of an absorbent sheet in which absorbent materials are arranged in two layers.

In addition, it is easier to miniaturize the absorbent sheet manufacturing apparatus compared to forming the first and second composite sheets while using conveyors instead of first and second conveyance rolls to convey the first and second continuous sheets along a linear path.

Preferably, between the third step and the fourth step, the first composite sheet is conveyed by supporting the first composite sheet on a first support surface and moving the first support surface while being sucked onto the first support surface.

In this case, between the third and fourth steps, the first absorbent material in the first composite sheet is prevented from being displaced in the planar direction even when the first composite sheet is conveyed at high speed. This allows for increased conveyance speed, improving production efficiency. Furthermore, the apparatus for manufacturing the first composite sheet and the apparatus for manufacturing the second composite sheet can be spaced apart, facilitating simplification and standardization of the apparatus configuration.

Preferably, between the third step and the fourth step, the first composite sheet is wound around and supported by a first reversing roll that is disposed adjacent to the first conveying roll and rotates in a direction opposite to that of the first conveying roll, and is conveyed while being supported.

In this case, by winding the first composite sheet around the first reversing roll, the pressure bonding between the first and second continuous sheets of the first composite sheet is strengthened, thereby suppressing the movement of the first absorbent material in the first composite sheet in the planar direction.

More preferably, the first composite sheet discharged from the first reversing roll is supported horizontally and conveyed horizontally using a first conveyor, and the first composite sheet is supplied to the second conveyance roll.

In this case, movement of the first absorbent material in the first composite sheet in the planar direction can be suppressed in the section from the first reversing roll to the second conveyance roll.

Preferably, between the sixth step and the seventh step, the second composite sheet is conveyed by supporting the second composite sheet on a second support surface and moving the second support surface while being sucked onto the second support surface.

In this case, between the sixth and seventh steps, the first and second absorbent materials in the second composite sheet are prevented from being displaced in the planar direction even when the second composite sheet is conveyed at high speed. Therefore, it is possible to improve production efficiency by increasing the conveying speed. Furthermore, the apparatus for manufacturing the second composite sheet and the press apparatus can be arranged with a gap between them.

Preferably, between the sixth step and the seventh step, the second composite sheet is wound around and supported by a second reversing roll that is disposed adjacent to the second conveying roll and rotates in a direction opposite to that of the second conveying roll, and is conveyed while being supported.

In this case, by winding the second composite sheet around the second reversing roll, the pressure bonding between the first to third continuous sheets of the second composite sheet is strengthened, thereby suppressing the movement of the first and second absorbent materials in the second composite sheet in the planar direction.

More preferably, the second composite sheet discharged from the second reversing roll is supported horizontally and conveyed horizontally using a second conveyor, and the second composite sheet is supplied to the press apparatus.

In this case, in the section from the second reversing roll to the second conveyance roll, movement of the first and second absorbent materials in the second composite sheet in the planar direction can be suppressed.

Preferably, between the third step and the fourth step, the first composite sheet, which is wound around and conveyed by the first reversing roll, is pressed toward the first reversing roll by a first belt and/or a first press roll.

In this case, it is possible to suppress the movement of the first absorbent material in the first composite sheet in the planar direction.

Preferably, between the sixth step and the seventh step, the second composite sheet, which is wound around and conveyed by the second reversing roll, is pressed toward the second reversing roll by a second belt and/or a second press roll.

In this case, it is possible to suppress the movement of the second absorbent material in the second composite sheet in the planar direction.

Preferably, the second composite sheet is compressed in the thickness direction using the second belt and/or the second press roll and the second reversing roll as the press apparatus.

In this case, the configuration of the apparatus for manufacturing the absorbent sheet can be simplified.

In order to solve the above problems, the present invention also provides an apparatus for manufacturing an absorbent sheet used for an absorbent article, configured as follows.

The apparatus for manufacturing an absorbent sheet used for an absorbent article is provided with: (a) a first composite sheet forming apparatus that is supplied with a first continuous sheet and a second continuous sheet and forms a first composite sheet having a first absorbent material disposed between the first continuous sheet and the second continuous sheet; (b) a second composite sheet forming apparatus that is supplied with the first composite sheet and a third continuous sheet and forms a second composite sheet having a second absorbent material disposed between the first composite sheet and the third continuous sheet; and (c) a press apparatus that compresses the second composite sheet in the thickness direction.

According to the above configuration, an absorbent sheet in which absorbent materials are arranged in two layers can be manufactured using three continuous sheets, which reduces the number of continuous sheets by one compared to when an absorbent sheet in which absorbent materials are arranged in two layers is manufactured using four continuous sheets, thereby reducing the manufacturing cost of an absorbent sheet in which absorbent materials are arranged in two layers.

Preferably, the apparatus for manufacturing an absorbent sheet is further provided with (d) a first conveyor that is disposed between the first composite sheet forming apparatus and the second composite sheet forming apparatus, has a first conveying surface that supports the first composite sheet and moves while sucking the first composite sheet, and conveys the first composite sheet toward the second composite sheet forming apparatus.

In this case, the first absorbent material in the first composite sheet being conveyed by the first conveyor is prevented from being displaced in the planar direction even when the first composite sheet is conveyed at high speed. Therefore, it is possible to improve production efficiency by increasing the convey speed. Furthermore, the first composite sheet forming apparatus and the second composite sheet forming apparatus can be arranged with a gap between them, which facilitates simplification and standardization of the configurations of the first composite sheet forming apparatus and the second composite sheet forming apparatus.

Preferably, the first composite sheet forming apparatus is provided with (i) a first conveyance roll around which the first continuous sheet is wound and which conveys the first continuous sheet; (ii) a first absorbent material supplying apparatus that supplies the first absorbent material to the first continuous sheet being wound around and conveyed by the first conveyance roll; (iii) a first guide roll that guides the second continuous sheet so as to overlap the second continuous sheet onto the first continuous sheet so as to cover the first absorbent material supplied to the first continuous sheet, to form the first composite sheet; and (iv) a first reversing roll disposed adjacent to the first conveyance roll, around which the first composite sheet is wound so as to support the second continuous sheet of the first composite sheet, and which rotates in a direction opposite to that of the first conveyance roll to convey the first composite sheet: and configured such that the first composite sheet is sucked onto a first support surface on which the first composite sheet is supported.

In this case, the movement of the first absorbent material in the first composite sheet in the planar direction can be suppressed, and the production efficiency can be improved by increasing the conveyance speed. Furthermore, the first composite sheet forming apparatus can be made smaller than when the first continuous sheet is conveyed along a linear path.

Preferably, the apparatus for manufacturing an absorbent sheet is further provided with (e) a second conveyor that is disposed between the second composite sheet forming apparatus and the press apparatus, has a second conveying surface that supports the second composite sheet and moves while sucking the second composite sheet, and conveys the second composite sheet.

In this case, the first and second absorbent materials in the second composite sheet being conveyed by the second conveyor are prevented from being displaced in the planar direction even when the second composite sheet is conveyed at high speed. This allows for improved production efficiency by increasing the conveying speed. Furthermore, the second composite sheet forming apparatus and the press apparatus can be arranged with a gap between them, facilitating simplification and standardization of the apparatus configuration.

Preferably, the second composite sheet forming apparatus is provided with (i) a second conveyance roll around which the first composite sheet is wound and which conveys the second composite sheet; (ii) a second absorbent material supplying apparatus that supplies the second absorbent material to the first composite sheet that is wound around and conveyed by the second conveyance roll; (iii) a second guide roll that guides the third continuous sheet so as to overlap the third continuous sheet onto the first composite sheet so as to cover the second absorbent material supplied to the second composite sheet, to form the second composite sheet; and (iv) a second reversing roll disposed adjacent to the second conveyance roll, around which the second composite sheet is wound so as to support the third continuous sheet of the second composite sheet, and which rotates in a direction opposite to that of the first conveyance roll to convey the second composite sheet: and configured such that the second composite sheet is sucked onto a second support surface on which the second composite sheet is supported.

In this case, the movement of the first and second absorbent materials in the second composite sheet in the planar direction can be suppressed, and the production efficiency can be improved by increasing the conveying speed. Furthermore, the second composite sheet forming apparatus can be made smaller than when the second composite sheet is conveyed along a linear path.

Preferably, the apparatus for manufacturing an absorbent sheet is further provided with a first belt and/or a first press roll that is disposed opposite the first reversing roll and presses the first composite sheet, which is wound around and conveyed by the first reversing roll, toward the first reversing roll.

In this case, it is possible to suppress the movement of the first absorbent material in the first composite sheet in the planar direction.

Preferably, the apparatus for manufacturing an absorbent sheet is further provided with a second belt and/or a second press roll that is disposed opposite the second reversing roll and presses the second composite sheet, which is wound around and conveyed by the second reversing roll, toward the second reversing roll.

In this case, it is possible to suppress the movement of the second absorbent material in the second composite sheet in the planar direction.

Preferably, the second belt and/or the second press roll and the second reversing roll are configured as the press apparatus that compresses the second composite sheet in the thickness direction.

In this case, the configuration of the apparatus for manufacturing an absorbent sheet can be simplified.

### EFFECTS OF THE INVENTION

According to the present invention, it is possible to reduce the manufacturing cost of an absorbent sheet in which absorbent materials are arranged in two layers.

### BRIEF DESCRIPTION OF THE DRAWINGS

[Fig. 1] Fig. 1 is a schematic view showing the overall configuration of an apparatus for manufacturing an absorbent sheet (Example 1).
[Fig. 2] Fig. 2 is an enlarged schematic view showing the configuration of the main parts of an apparatus for manufacturing an absorbent sheet (Example 1).
[Fig. 3] Fig. 3 is an enlarged schematic view showing the configuration of the main parts of an apparatus for manufacturing an absorbent sheet (Example 1).
[Fig. 4] Fig. 4(a) is a schematic exploded cross-sectional view of the absorbent body, Fig. 4(b) is a plan view taken along line B-B in Fig. 4(a), and Fig. 4(c) is a plan view taken along line C-C in Fig. 4(a).
[Fig. 5] Fig. 5(a) is a schematic side view of a partial cross section of the first press roll on the upstream side, and Fig. 5(b) is a schematic side view of the first press roll on the downstream side (Example 1).
[Fig. 6] Fig. 6 is a schematic cross-sectional view of a second composite sheet in the width direction (Example 1).
[Fig. 7] Fig. 7 is a schematic view of an apparatus for manufacturing an absorbent sheet (Conventional Example 1).

### MODE FOR CARRYING OUT THE INVENTION

Hereinafter, embodiments of the present invention will be described with reference to the drawings.

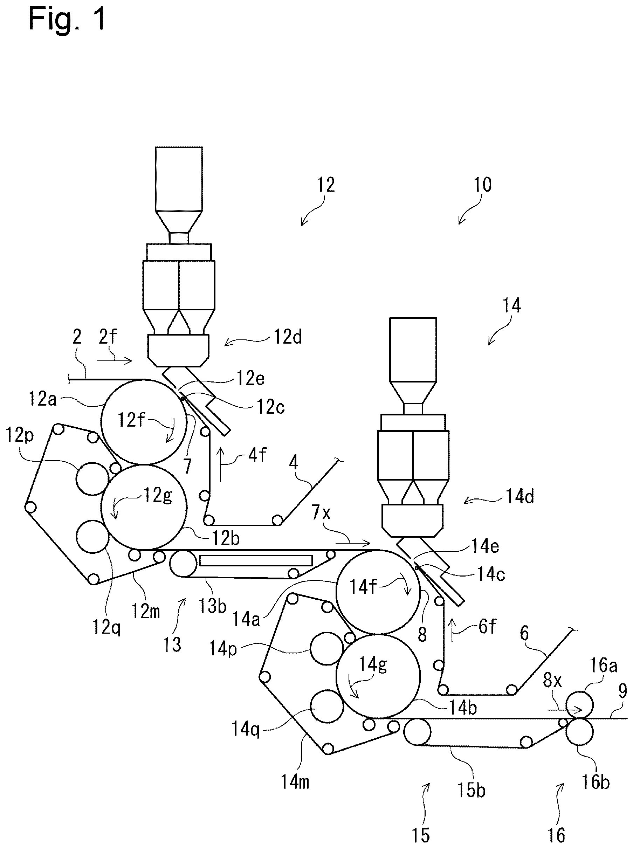

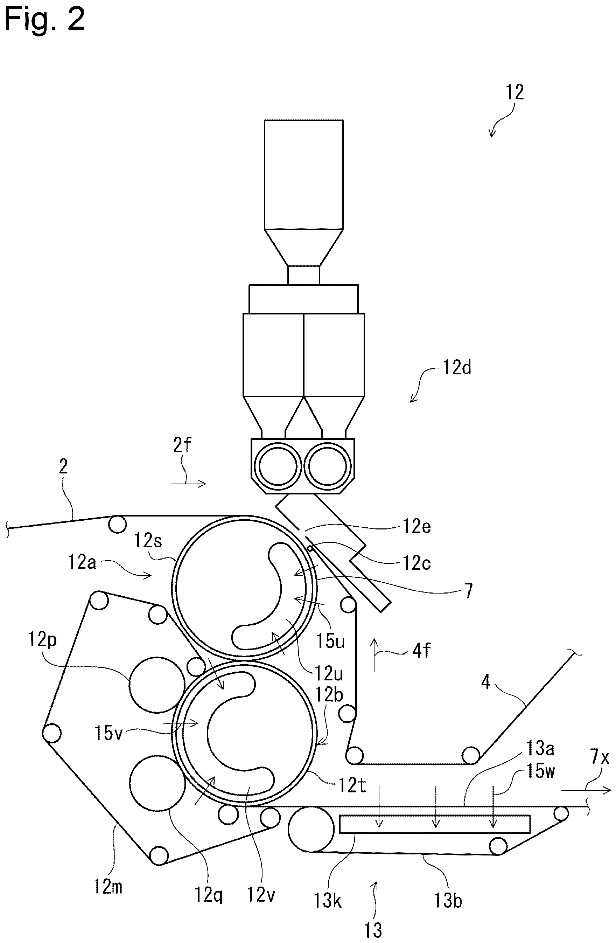

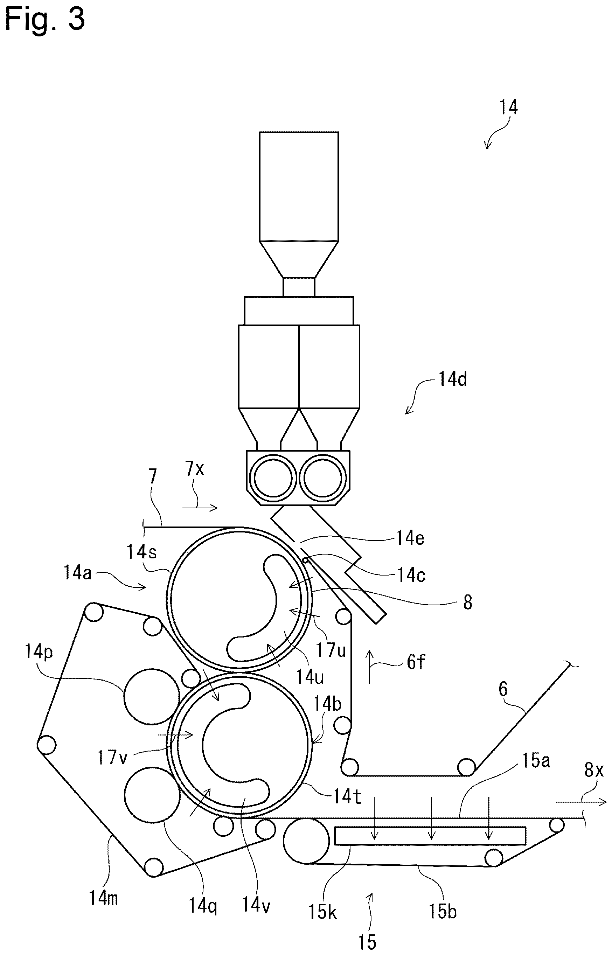

<Example 1> The apparatus and method for manufacturing an absorbent sheet of Example 1 will be described with reference to Figs. 1 to 4. Fig. 1 is a schematic view showing the overall configuration of the apparatus for manufacturing an absorbent sheet 10. Figs. 2 and 3 are enlarged schematic views showing the configuration of the main parts of the apparatus for manufacturing an absorbent sheet 10.

As shown in Figs. 1 to 3, the apparatus for manufacturing an absorbent sheet 10 includes a first composite sheet forming apparatus 12, a first conveyor 13, a second composite sheet forming apparatus 14, a second conveyor 15, and a press apparatus 16.

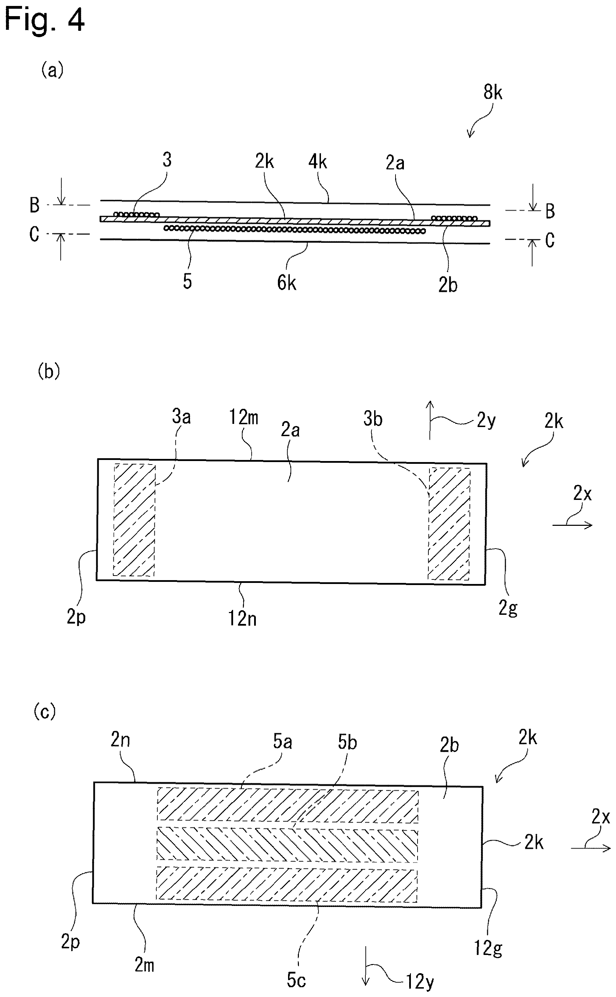

The first composite sheet forming apparatus 12 is supplied with first and second continuous sheets 2, 4 such as nonwoven fabric in the directions indicated by arrows 2f, 4f, and forms a first composite sheet 7 in which a first absorbent material 3 (see Fig. 4(a)) such as granular or powdered SAP is disposed between the first continuous sheet 2 and the second continuous sheet 4.

More specifically, the first composite sheet forming apparatus 12 has a first SAP supplying apparatus 12d, a first guide roll 12c, and a first reversing roll 12b arranged around a first conveyance roll 12a.

The first conveyance roll 12a has a cylindrical outer peripheral surface 12s around which the first continuous sheet 2 is wound, and rotates in the direction indicated by an arrow 12f to convey the first continuous sheet 2.

The first SAP supply apparatus 12d has an opening 12e facing the first continuous sheet 2 wound around the first conveyance roll 12a, and is configured so that the first absorbent material 3 drops from the opening 12e. The opening 12e opens and closes at appropriate times, thereby supplying the first absorbent material 3 so that the first absorbent material 3 is disposed in a predetermined region on the first continuous sheet 2 which is wound around and conveyed by the first conveyance roll 12a.

The first guide roll 12c guides the second continuous sheet 4 so that it overlaps the first continuous sheet 2 to which the first absorbent material 3 has been supplied and covers the first absorbent material 3. In this way, a first composite sheet 7 is formed.

The first reversing roll 12b is disposed adjacent to the first conveyance roll 12a, and the first composite sheet 7 is wound around it so as to support the second continuous sheet 4 of the first composite sheet 7. The first reversing roll 12b rotates in a direction opposite to that of the first conveyance roll 12a as indicated by arrow 12g, and conveys the first composite sheet 7.

As shown in Fig. 2, the outer surface 12s of the first conveying roll 12a and the outer surface 12t of the first reversing roll 12b are first support surfaces 12s, 12t that support the first composite sheet 7 and are configured to suck the first composite sheet 7.

For example, first suction boxes 12u, 12v connected to a vacuum source or a reduced pressure source are arranged inside the first conveying roll 12a and the first reversing roll 12b, and through holes (not shown) communicating with the first suction boxes 12u, 12v are formed in the first support surfaces 12s, 12t, so that the first composite sheet 7 is sucked onto the first support surfaces 12s, 12t by air flow in the direction indicated by arrows 15u, 15v.

By sucking the first composite sheet 7 onto the first support surfaces 12s, 12t, the first absorbent material 3 in the first composite sheet 7 is prevented from being displaced in the planar direction even when the first composite sheet 7 is conveyed at high speed. Therefore, it is possible to increase the conveying speed and improve the production efficiency of the absorbent sheet 9.

A first belt 12m may be disposed around the first reversing roll 12b so as to be movable along the first reversing roll 12b, and the first composite sheet 7 may be conveyed while being sandwiched between the first reversing roll 12b and the first belt 12m. In this case, the pressure bonding between the first continuous sheet 2 and the second continuous sheet 4 of the first composite sheet 7 is strengthened, further suppressing the movement of the first absorbent material 3 in the first composite sheet 7 in the planar direction. The first belt 12m may be non-breathable or may be breathable enough to be sucked by the first reversing roll 12b.

Instead of the first belt 12m, or together with the first belt 12m, first press rolls 12p and 12q may be arranged to face the first reversing roll 12b, and the first composite sheet 7 wound around and conveyed by the first reversing roll 12b may be pressed toward the first reversing roll 12b by the first press rolls 12p and 12q. The number of first press rolls 12p and 12q may be one, or three or more.

When the first press rolls 12p, 12q are arranged together with the first belt 12m, the first press rolls 12p, 12q are configured to press the first composite sheet 7 toward the first reversing roll 12b via the first belt 12m.

By providing the first press rolls 12p, 12q, it is possible to suppress the shift between the first continuous sheet 2 and the second continuous sheet 4 of the first composite sheet 7, thereby further suppressing the movement of the first absorbent material 3 in the first composite sheet 7 in the planar direction.

The first conveyor 13 is disposed between the first composite sheet forming apparatus 12 and the second composite sheet forming apparatus 14, and conveys the first composite sheet 7 discharged from the first reversing roll 12b of the first composite sheet forming apparatus 12 toward the second composite sheet forming apparatus 14.

The first conveyor 13 is configured such that a breathable first endless belt 13b moves in a circulating manner, and a third suction box 13k connected to a vacuum source or a reduced pressure source is disposed inside the first endless belt 13b, and is configured such that the first composite sheet 7 is sucked onto the upper surface 13a of the first endless belt 13b by air flow in the direction indicated by arrow 15W. The upper surface 13a of the first endless belt 13b is the first convey surface 13a that horizontally supports the first composite sheet 7 and moves it horizontally while sucking it.

The second composite sheet forming apparatus 14 is supplied with the first composite sheet 7 discharged from the first conveyor 13 in the direction indicated by the arrow 7x and a third continuous sheet 6 such as a nonwoven fabric, and forms a second composite sheet 8 in which a second absorbent material 5 (see Fig. 4(a)) such as granular or powdered SAP is disposed between the first composite sheet 7 and the third continuous sheet 6.

The second composite sheet forming apparatus 14 has the same configuration as the first composite sheet forming apparatus 12, and a second SAP supply apparatus 14d, a second guide roll 14c, and a second reversing roll 14b are arranged around the second conveying roll 14a.

Second suction boxes 14u and 14v are arranged inside the second conveying roll 14a and the second reversing roll 14b, and are configured to suck the second composite sheet 8 onto the second support surfaces 14s and 14t of the second conveying roll 14a and the second reversing roll 14b by air flow in the direction indicated by arrows 17u and 17v.

A second belt 14m may be disposed around the second reversing roll 14b so as to be movable along the second reversing roll 14b, similar to the first reversing roll 12b, so that the second composite sheet 8 is conveyed while being sandwiched between the second reversing roll 14b and the second belt 14m. In this case, the pressure bonding between the first composite sheet 7 and the third continuous sheet 6 of the second composite sheet 8 is strengthened, further suppressing the movement of the second absorbent material 5 in the plane direction within the second composite sheet 8. The second belt 14m may be non-breathable or may be breathable enough to be sucked by the second reversing roll 14b.

In addition to or instead of the second belt 14m, second press rolls 14p and 14q may be disposed opposite the second reversing roll 14b to press the second composite sheet 8 toward the second reversing roll 14b. In this case, it is possible to further suppress the movement of the second absorbent material 5 in the second composite sheet 8 in the planar direction. The number of second press rolls 14p and 14q may be one, or three or more.

The second composite sheet 8 discharged from the second reversing roll 14b of the second composite sheet forming apparatus 14 is conveyed by the second conveyor 15 toward the press apparatus 16.

The second conveyor 15 has the same configuration as the first conveyor 13, and has a second endless belt 15b that is breathable and moves in a circular motion. A fourth suction box 15k connected to a vacuum source or reduced pressure source is arranged inside the second endless belt 15b, and is configured to suck the second composite sheet 8 onto the upper surface 15a of the second endless belt 15b, i.e., the second conveying surface 15a.

The second composite sheet 8 discharged from the second conveyor 15 in the direction indicated by the arrow 8x is compressed in the thickness direction when passing between a pair of rolls 16a, 16b of the press apparatus 16. In this way, the absorbent sheet 9 is formed.

The absorbent sheet 9 is cut to a predetermined size in a subsequent process to form, for example, individual pieces of absorbent body 8k as shown in Fig. 4. The absorbent body 8k is placed in an absorbent article such as a disposable diaper or a sanitary napkin.

Fig. 4(a) is a schematic exploded cross-sectional view of the absorbent body 8k, Fig. 4(b) is a plan view taken along line B-B in Fig. 4(a), and Fig. 4(c) is a plan view taken along line C-C in Fig. 4(a).

As shown in Fig. 4(a), a first absorbent material 3 is disposed on one major surface 2a of a first sheet 2k formed from a first continuous sheet 2, and a second absorbent material 5 is disposed on the other major surface 2b of the first sheet 2k. The first absorbent material 3 is sandwiched between the first sheet 2k and a second sheet 4k formed from a second continuous sheet 4. The second absorbent material 5 is sandwiched between the first sheet 2k and a third sheet 6k formed from a third continuous sheet 6. That is, the first absorbent material 3 is disposed in a first layer between the first sheet 2k and the second sheet 4k, and the second absorbent material 5 is disposed in a second layer between the first sheet 2k and the third sheet 6k. The first and second absorbent materials 3, 5 can be disposed in various patterns.

For example, as shown in Fig. 4(b), the first absorbent material 3 is arranged in two regions 3a and 3b on both sides in the longitudinal direction, and as shown in Fig. 4(c), the second absorbent material 5 is arranged in three regions 5a, 5b, and 5c at the longitudinal center, spaced apart and extending in the longitudinal direction.

By using the apparatus for manufacturing an absorbent sheet 10 described above, an absorbent sheet 9 in which absorbent materials 3 and 5 are arranged in two layers can be manufactured using three continuous sheets 2, 4, and 6. By forming two composite sheets each having an absorbent material sandwiched between two continuous sheets and then overlapping the two composite sheets, it is possible to reduce the number of continuous sheets by one compared to when an absorbent sheet in which absorbent materials are arranged in two layers is manufactured using four continuous sheets. Therefore, by using the apparatus for manufacturing an absorbent sheet 10, the manufacturing cost of the absorbent sheet 9 can be reduced.

By conveying the first composite sheet 7 while sucking it on the first conveyor 13, the first absorbent material 3 in the first composite sheet 7 during convey is prevented from being displaced in the planar direction even when the first composite sheet 7 is conveyed at high speed. This makes it possible to improve production efficiency by increasing the conveying speed. Furthermore, the first composite sheet forming apparatus 12 and the second composite sheet forming apparatus 14ccan be arranged with a gap between them, which facilitates simplification and standardization of the configurations of the first composite sheet forming apparatus 12 and the second composite sheet forming apparatus 14.

Similarly, by conveying the second composite sheet 8 while sucking it on the second conveyor 15, the first and second absorbent materials 3 and 5 within the second composite sheet 8 are prevented from being displaced in the planar direction even when the second composite sheet 8 is conveyed at high speed. This makes it possible to improve production efficiency by increasing the conveying speed. Furthermore, the second composite sheet forming apparatus 14 and the press apparatus 16 can be arranged with a gap between them.

When the first composite sheet forming apparatus 12 is composed of a first conveying roll 12a, a first reversing roll 12b, etc., and the first composite sheet 7 is conveyed while being sucked onto the first support surfaces 12s, 12t, it is possible to suppress the movement of the first absorbent material 3 in the first composite sheet 7 in the planar direction, thereby improving production efficiency by increasing the conveying speed. Furthermore, the first composite sheet forming apparatus 12 can be made smaller than when the first composite sheet 7 is conveyed along a linear path.

Similarly, if the second composite sheet forming apparatus 14 is composed of a second conveying roll 14a, a second reversing roll 14b, etc., and the second composite sheet 8 is conveyed while being sucked onto the second support surfaces 14s, 14t, it is possible to suppress the movement of the first and second absorbent materials 3, 5 in the second composite sheet 8 in the planar direction, thereby improving production efficiency by increasing the conveying speed. Furthermore, the second composite sheet forming apparatus 14 can be made smaller than when the second composite sheet 8 is conveyed along a linear path.

Next, the first press rolls 12p, 12q and the second press rolls 14p, 14q will be described in more detail.

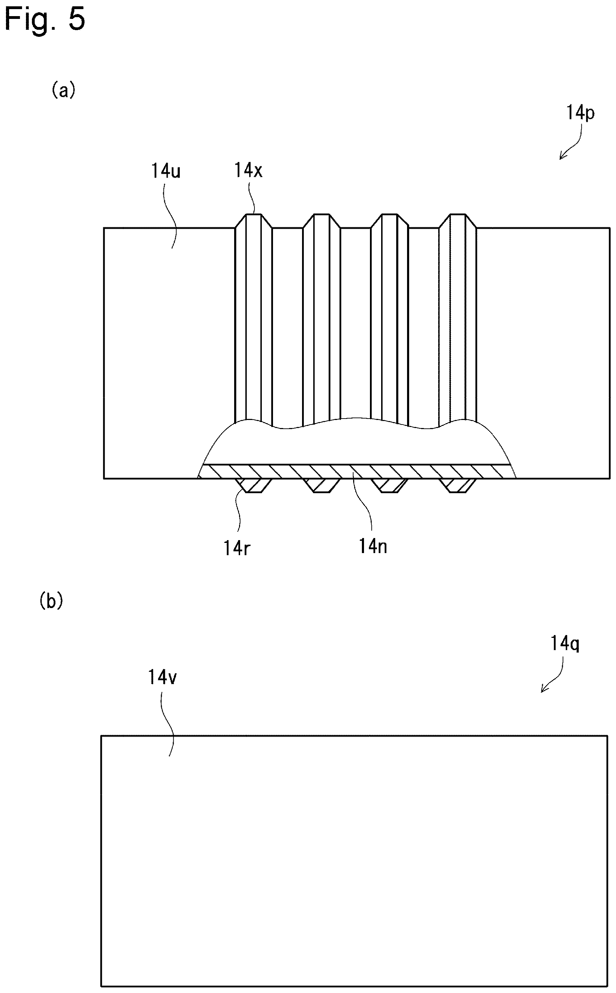

Fig. 5(a) is a schematic partial cross-sectional side view of the upstream second press roll 14p. As shown in Fig. 5(a), the upstream second press roll 14p has four circumferentially extending ridges 14x formed on a cylindrical outer peripheral surface 14u. The upstream second press roll 14p has an outer peripheral member 14n to which elongated members 14r for forming the ridges 14x are fixed by welding or the like, for example.

Fig. 5(b) is a schematic side view of the downstream second press roll 14q. As shown in Fig. 5(b), the downstream second press roll 14q has a cylindrical outer peripheral surface 14v, and the outer peripheral surface 14v is not formed with any protrusions or ridges.

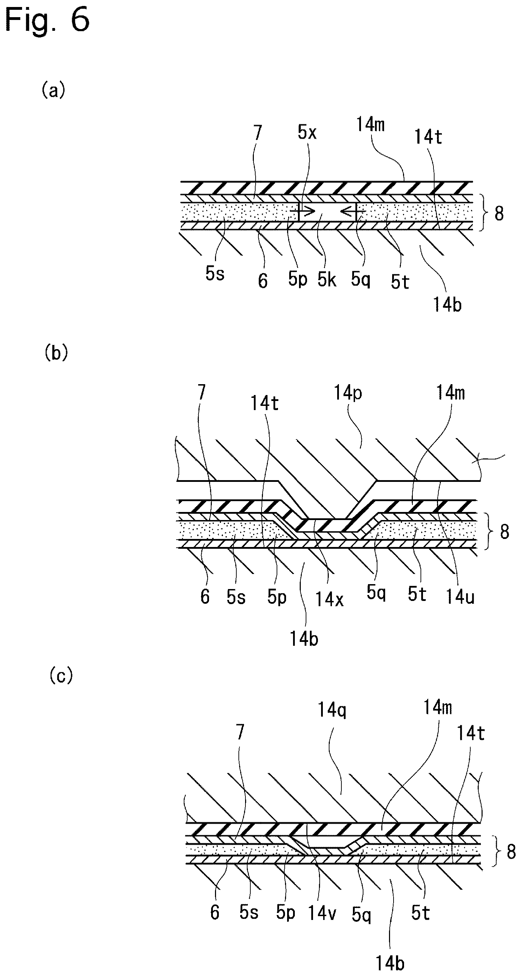

Fig. 6 is a schematic cross-sectional view in the width direction of the second composite sheet 8 when compressed by the second press rolls 14p and 14q.

As shown in Fig. 6(a), the second composite sheet 8 is sandwiched between the second support surface 14t of the second reversing roll 14b and the second belt 14m.

Next, as shown in Fig. 6(b), the ridges 14x of the upstream second press roll 14p press the region 5k (see Fig. 6(a)) adjacent to the regions 5s and 5t where the second absorbent material 5 is arranged, thereby compressing the second absorbent materials 5p and 5q arranged near the boundary between the regions 5s and 5t.

Next, as shown in Fig. 6(c), the second composite sheet 8 is pressed across its entire width by a second press roll 14q on the downstream side.

At this time, the second absorbent material 5p, 5q arranged near the boundary line between the regions 5s, 5t does not move toward the outside of the regions 5s, 5t, so that a well-shaped slit (long, narrow gap) can be formed between the regions 5s, 5t where the second absorbent material 5 is arranged.

That is, if the second composite sheet 8 in the state shown in Fig. 6(a) was immediately pressed across the entire width using a roll with a cylindrical outer surface, the second absorbent materials 5p, 5q arranged near the boundary line of the regions 5s, 5t would move toward the outside of the regions 5s, 5t as indicated by the arrow 5x, which would likely narrow the slit width or distort the slit shape. In contrast, if the second absorbent materials 5p, 5q arranged near the boundary line of the regions 5s, 5t is first compressed using the upstream second press roll 14p, when pressed across the entire width using the downstream second press roll 14q, the second absorbent materials 5p, 5q near the boundary line of the regions 5s, 5t would not move toward the outside of the regions 5s, 5t. This allows for the formation of slits with a good shape.

That is, the upstream second press roll 14p is used to form the slits, and the downstream second press roll 14q is used to press the entire second composite sheet 8 after the slits have been formed.

Note that Fig. 5(b) illustrates a case where the outer surface 14u of the upstream second press roll 14p does not press the second belt 14m, but the outer surface 14u of the upstream second press roll 14p may press the regions 14s and 14t of the second composite sheet 8 via the second belt 14m.

As shown in Fig. 4(b), when forming an absorbent sheet 9 in which the first absorbent material 3 is arranged in two spaced-apart regions 3a, 3b, the upstream first press roll 12p has ridges extending in the width direction formed at intervals in the circumferential direction on the cylindrical outer peripheral surface of the upstream first press roll 12p so as to press the area near the boundary line between the regions 3a and 3b, and/or, circumferentially extending protrusions formed at intervals in the axial direction on the outer peripheral surface of the upstream first press roll 12p so as to press the areas near the boundary lines on both sides of the width direction of the regions 3a and 3b. The downstream first press roll 12q, like the downstream second press roll 14q, has no ridges or protrusions formed on its cylindrical outer peripheral surface.

Although not shown, the first press rolls 12p, 12q may be configured similarly to the second press rolls 14p, 14q, with the upstream first press roll 12p being used to form the slits and the downstream first press roll 12q being used to press the entire first composite sheet 7 after the slits have been formed.

Next, a method for manufacturing the absorbent sheet 9 using the apparatus for manufacturing an absorbent sheet 10 will be described. The method for manufacturing the absorbent sheet 9 includes first to seventh steps.

In the first step, the first continuous sheet 2 is wound around and conveyed by the rotating first conveyance roll 12a of the first composite sheet forming apparatus 12.

In the second step, the first SAP supplying apparatus 12d of the first composite sheet forming apparatus 12 supplies the first absorbent material 3 to the first continuous sheet 2 that is wound around and conveyed by the first conveyance roll 12a.

In the third step, the first guide roll 12c of the first composite sheet forming apparatus 12 overlaps the second continuous sheet 4 onto the first continuous sheet 2 so as to cover the first absorbent material 3 supplied to the first continuous sheet 2, thereby forming a first composite sheet 7 in which the first absorbent material 3 is sandwiched between the first continuous sheet 2 and the second continuous sheet 4.

In the fourth step, the first composite sheet 7 is wound around and conveyed by a rotating second conveyance roll 14a of a second composite sheet forming apparatus 14.

In the fifth step, the second SAP supply apparatus 14d of the second composite sheet forming apparatus 14 supplies the second absorbent material 5 to the first composite sheet 7 being wound around and conveyed by the second conveyance roll 14a.

In the sixth step, the second guide roll 14c of the second composite sheet forming apparatus 14 overlaps the third continuous sheet 6 onto the first composite sheet 7 so as to cover the second absorbent material 5 supplied to the first composite sheet 7, thereby forming a second composite sheet 8 in which the first absorbent material 3 is sandwiched between the first continuous sheet 2 and the second continuous sheet 4, and the second SAP 4 is sandwiched between the second continuous sheet 4 and the third continuous sheet 6.

In the seventh step, the second composite sheet 8 is compressed in the thickness direction using a press apparatus 16 to form the absorbent sheet 9.

Preferably, between the third and fourth steps, the first composite sheet 7 is supported by the first support surfaces 12s, 12t and conveyed by moving the first support surfaces 12s, 12t while being sucked onto the first support surfaces 12s, 12t.

Specifically, between the third and fourth steps, the first composite sheet 7 is wound around and supported by a first reversing roll 12b, which is disposed adjacent to the first conveying roll 12a and rotates in a direction opposite to that of the first conveying roll 12a, and is conveyed while being supported. In this case, by winding the first composite sheet 7 around the first reversing roll 12b, the pressure bonding between the first and second continuous sheets 2 and 4 of the first composite sheet 7 is strengthened, and movement of the first absorbent material 3 in the first composite sheet 7 in the planar direction can be suppressed.

Furthermore, the first composite sheet 7 discharged from the first reversing roll 12b is supported horizontally and conveyed horizontally using the first conveyor 13, and the first composite sheet 7 is supplied to the second conveyance roll 14a. In this case, movement of the first absorbent material 3 in the first composite sheet 7 in the planar direction can be suppressed in the section from the first reversing roll 12b to the second conveyance roll 14a.

Preferably, the first composite sheet 7 is conveyed while being sandwiched between the first reversing roll 12b and the first belt 12m, using a first belt 12m arranged movably along the first reversing roll 12b. In this case, the pressure bonding between the first continuous sheet 2 and the second continuous sheet 4 of the first composite sheet 7 is further strengthened, and movement of the first absorbent material 3 in the first composite sheet 7 in the planar direction can be further suppressed.

Preferably, the first composite sheet 7, which is wound around and conveyed by the first reversing roll 12b, is pressed toward the first reversing roll 12b using first press rolls 12p and 12q disposed opposite the first reversing roll 12b. In this case, it is possible to suppress misalignment between the first continuous sheet 2 and the second continuous sheet 4 of the first composite sheet 7, and further suppress movement of the first absorbent material 3 in the first composite sheet 7 in the planar direction.

Preferably, between the sixth and seventh steps, the second composite sheet 8 is supported by the second support surfaces 14s, 14t and conveyed by moving the second support surfaces 14s, 14t while being sucked onto the second support surfaces 14s, 14t.

More specifically, between the sixth and seventh steps, the second composite sheet 8 is wound around and supported by a second reversing roll 14b, which is disposed adjacent to the second conveyance roll 14a and rotates in a direction opposite to that of the second conveyance roll 14a, and is conveyed while being supported. In this case, by winding the second composite sheet 8 around the second reversing roll 14b, the pressure bonding between the first to third continuous sheets 2, 4, and 6 of the second composite sheet 8 is strengthened, and movement of the first and second absorbent materials 3 and 5 in the plane direction within the second composite sheet 8 can be suppressed.

Furthermore, the second composite sheet 8 discharged from the second reversing roll 14b is supported horizontally and conveyed horizontally using the second conveyor 15, and the second composite sheet 8 is supplied to the press apparatus 16. In this case, in the section from the second reversing roll 14b to the press apparatus 16, movement of the first and second absorbent materials 3, 5 in the second composite sheet 8 in the planar direction can be suppressed.

Preferably, a second belt 14m is disposed movably along the second reversing roll 14b, and the second composite sheet 8 is conveyed while being sandwiched between the second reversing roll 14b and the second belt 14m. In this case, the pressure bonding between the first to third continuous sheets 2, 4, 6 of the second composite sheet 8 is further strengthened, and the movement of the first and second absorbent materials 3, 5 in the second composite sheet 8 in the planar direction can be further suppressed.

Preferably, second press rolls 14p and 14q disposed opposite the second reversing roll 14b are used to press the second composite sheet 8, which is wound around and conveyed by the second reversing roll 14b, toward the second reversing roll 14b. In this case, it is possible to suppress misalignment between the first to third continuous sheets 2, 4, and 6 of the second composite sheet 8, and further suppress movement of the first and second absorbent materials 3 and 5 in the second composite sheet 8 in the planar direction.

Next, modification examples of the first embodiment will be described.

### <Modification Example 1>

The absorbent sheet 9 may be formed without using the first conveyor 13 and/or the second conveyor 15.

### <Modification Example 2>

The absorbent sheet may be formed without using the first reversing roll 12b and/or the second reversing roll 14b.

If the first reversing roll 12b is not used, the second continuous sheet 4 of the first composite sheet 7 can be configured to be supported by the conveying roll 14a of the second composite sheet forming apparatus 14, and a second composite sheet 8 can be formed in which the second absorbent material 5 is sandwiched between the first continuous sheet 2 and the third continuous sheet 6, rather than between the second continuous sheet 4 and the third continuous sheet 6.

### <Modification Example 3>

The suction during the convey of the first composite sheet 7 and/or the suction during the convey of the second composite sheet 8 may be eliminated in whole or in part.

### <Modification Example 4>

Instead of using the first conveyance roll 12a and/or the second conveyance roll 14a, the first continuous sheet 2 and/or the first composite sheet 7 may be conveyed along a linear path using a conveyor or the like to form the first composite sheet 7 and/or the second composite sheet 8.

### <Modification Example 5>

A first composite sheet 7 may be formed by bonding a first continuous sheet 2 and a second continuous sheet 4 to each other. For example, a second continuous sheet 4 coated with an adhesive may be overlapped onto and bonded to a first continuous sheet 2 to which a first absorbent material 3 has been supplied. An adhesive may also be applied to the first continuous sheet 2 to which the first absorbent material 3 has been supplied, thereby bonding the first continuous sheet 2 and the second continuous sheet 4 to each other. It is also possible to apply an adhesive to the first continuous sheet 2, then supply the first absorbent material 3, and bond the first continuous sheet 2 and the second continuous sheet 4 to each other.

In this case, it is preferable that the first reversing roll 12b and/or the first press rolls 12p, 12q have a heating function and heat the first composite sheet 7. For example, a heater may be provided inside or around the first reversing roll 12b and/or the first press rolls 12p, 12q to heat the first composite sheet 7 so that the temperature of the adhesive in the first composite sheet 7 is equal to or higher than the temperature at which the adhesive is applied. Heating facilitates the penetration of the adhesive in the first composite sheet 7 into the periphery of the first absorbent material 3 sandwiched between the first continuous sheet 2 and the second continuous sheet 4, thereby preventing the first absorbent material 3 from shifting out of position. As a result, it becomes easier to precisely position the first absorbent material 3 at the desired position between the first continuous sheet 2 and the second continuous sheet 4.

### <Modification Example 6>

A second continuous sheet 8 may be formed by bonding a first composite sheet 7 and a third continuous sheet 6 to each other. For example, a third continuous sheet 6 coated with an adhesive may be overlapped onto and bonded to a first composite sheet 7 to which a second absorbent material 5 has been supplied. An adhesive may also be applied to the first composite sheet 7 to which the second absorbent material 5 has been supplied, thereby bonding the first composite sheet 7 and the third continuous sheet 6 to each other. It is also possible to apply an adhesive to the first composite sheet 7, then supply the second absorbent material 5, and bond the first composite sheet 7 and the third continuous sheet 6 to each other.

In this case, it is preferable that the second reversing roll 14b and/or the second press rolls 14p, 14q have a heating function and heat the second composite sheet 8. For example, a heater may be provided inside or around the second reversing roll 14b and/or the second press rolls 14p, 14q to heat the second composite sheet 8 so that the temperature of the adhesive in the second composite sheet 8 is equal to or higher than the temperature at which the adhesive is applied. Heating facilitates the penetration of the adhesive in the second composite sheet 8 into the periphery of the second absorbent material 5 sandwiched between the first composite sheet 7 and the third continuous sheet 6, thereby preventing misalignment of the second absorbent material 5. As a result, it becomes easier to precisely position the second absorbent material 5 at the desired position between the first composite sheet 7 and the third continuous sheet 6.

### <Modification Example 7>

The press apparatus 16 of Example 1 may be omitted. In this case, the second belt 14m and/or the second press rolls 14p, 14q and the second reversing roll 14 are configured as a press apparatus that compresses the second composite sheet 8 in the thickness direction. That is, the second belt 14m and/or the second press rolls 14p, 14q and the second reversing roll 14b are used as the press apparatus to compress the second composite sheet 8 in the thickness direction to form the absorbent sheet 9.

It is also possible to appropriately combine the configurations of Example 1 and Modification Examples 1 to 7. For example, by combining the configurations of Modification Example 5 and Modification Example 6 to provide a heating function to both the first reversing roll 12b and/or the first press rolls 12p, 12q and the second reversing roll 14b and/or the second press rolls 14p, 14q, both the first absorbent material 3 and the second absorbent material 5 can be positioned accurately at the desired positions.

### <Summary>

As described above, the absorbent sheet 9 having two SAP layers can be manufactured using three continuous sheets 2, 4, and 6, which reduces the number of continuous sheets by one compared to when an absorbent sheet in which absorbent materials are arranged in two layers is manufactured using four continuous sheets, thereby reducing the manufacturing cost of the absorbent sheet in which absorbent materials are arranged in two layers.

The present invention is not limited to the above-described embodiment, but can be implemented with various modifications.

For example, the first to third continuous sheets 2, 4, and 6 may be joined together at appropriate locations by heat welding, ultrasonic bonding, etc. The first absorbent material 3 and the second absorbent material 5 may be the same or different from each other.

### DESCRIPTION OF REFERENCE NUMERALS

2 First continuous sheet
3 First absorbent material
4 Second continuous sheet
5 Second absorbent material
6 Third continuous sheet
7 First composite sheet
8 Second composite sheet
9 Absorbent sheet
10 Apparatus for manufacturing absorbent sheet
12 First composite sheet forming apparatus
12a First conveyance roll
12b First reversing roll
12c First guide roll
12d First SAP supply apparatus (first absorbent material supply apparatus)
12m First Belt
12p, 12q First press roll
12s, 12t First support surface
13 First conveyor
13a Upper surface (first conveying surface)
14 Second composite sheet forming apparatus
14a Second conveyance roll
14b Second reversing roll (press apparatus)
14c Second guide roll
14d Second SAP supply apparatus (second absorbent material supply apparatus)
14m Second belt (press apparatus)
14p, 14q Second press roll (press apparatus)
14s, 14t Second support surface
15 Second conveyor
15a Upper surface (second conveying surface)
16 Press apparatus

## Claims

1. A method for manufacturing an absorbent sheet used for an absorbent article, comprising:
a first step of winding a first continuous sheet around a rotating first conveyance roll and conveying the first continuous sheet;
a second step of supplying a first absorbent material to the first continuous sheet being wound around and conveyed by the first conveyance roll;
a third step of overlapping a second continuous sheet onto the first continuous sheet so as to cover the first absorbent material supplied to the first continuous sheet, thereby forming a first composite sheet in which the first absorbent material is sandwiched between the first continuous sheet and the second continuous sheet;
a fourth step of winding the first composite sheet around a rotating second conveyance roll and conveying the first composite sheet;
a fifth step of supplying a second absorbent material to the first composite sheet being wound around and conveyed by the first conveyance roll;
a sixth step of overlapping a third continuous sheet onto the first composite sheet so as to cover the second absorbent material supplied to the first composite sheet, thereby forming a second composite sheet in which the first absorbent material is sandwiched between the first continuous sheet and the second continuous sheet, and the second absorbent material is sandwiched between the first continuous sheet and the third continuous sheet or between the second continuous sheet and the third continuous sheet; and
a seventh step of compressing the second composite sheet in the thickness direction using a press apparatus.

2. The method for manufacturing an absorbent sheet according to claim 1, wherein, between the third step and the fourth step, the first composite sheet is conveyed by supporting the first composite sheet on a first support surface and moving the first support surface while being sucked onto the first support surface.

3. The method for manufacturing an absorbent sheet according to claim 1 or 2, wherein, between the third step and the fourth step, the first composite sheet is wound around and supported by a first reversing roll that is arranged adjacent to the first conveying roll and rotates in a direction opposite to that of the first conveying roll, and is conveyed while being supported.

4. The method for manufacturing an absorbent sheet according to claim 3, wherein the first composite sheet discharged from the first reversing roll is supported horizontally and conveyed horizontally using a first conveyor, thereby supplying the first composite sheet to the second conveyance roll.

5. The method for manufacturing an absorbent sheet according to claim 1, wherein, between the sixth step and the seventh step, the second composite sheet is conveyed by supporting the second composite sheet on a second support surface and moving the second support surface while being sucked onto the second support surface.

6. The method for manufacturing an absorbent sheet according to claim 1 or 5, wherein, between the sixth step and the seventh step, the second composite sheet is wound around and supported by a second reversing roll that is arranged adjacent to the second conveying roll and rotates in a direction opposite to that of the second conveying roll, and is conveyed while being supported.

7. The method for manufacturing an absorbent sheet according to claim 6, wherein the second composite sheet discharged from the second reversing roll is supported horizontally and conveyed horizontally using a second conveyor, thereby supplying the second composite sheet to the press apparatus.

8. The method for manufacturing an absorbent sheet according to claim 3, wherein, between the third step and the fourth step, the first composite sheet, which is wound around and conveyed by the first reversing roll, is pressed toward the first reversing roll by a first belt and/or a first press roll.

9. The method for manufacturing an absorbent sheet according to claim 6, wherein, between the sixth step and the seventh step, the second composite sheet, which is wound around and conveyed by the second reversing roll, is pressed toward the second reversing roll by a second belt and/or a second press roll.

10. The method for manufacturing an absorbent sheet according to claim 9, wherein the second composite sheet is compressed in the thickness direction using the second belt and/or the second press roll and the second reversing roll as the press apparatus.

11. An apparatus for manufacturing an absorbent sheet used for an absorbent article, comprising:
a first composite sheet forming apparatus that is supplied with a first continuous sheet and a second continuous sheet and forms a first composite sheet having a first absorbent material disposed between the first continuous sheet and the second continuous sheet;
a second composite sheet forming apparatus that is supplied with the first composite sheet and a third continuous sheet and forms a second composite sheet having a second absorbent material disposed between the first composite sheet and the third continuous sheet; and
a press apparatus that compresses the second composite sheet in the thickness direction.

12. The apparatus for manufacturing an absorbent sheet according to claim 11, further comprising:
a first conveyor that is disposed between the first composite sheet forming apparatus and the second composite sheet forming apparatus, has a first conveying surface that supports the first composite sheet and moves while sucking the first composite sheet, and conveys the first composite sheet toward the second composite sheet forming apparatus.

13. The apparatus for manufacturing an absorbent sheet according to claim 11 or 12, wherein
the first composite sheet forming apparatus comprises:
a first conveyance roll around which the first continuous sheet is wound and which conveys the first continuous sheet;
a first absorbent material supplying apparatus that supplies the first absorbent material to the first continuous sheet that is being wound around and conveyed by the first conveyance roll;
a first guide roll that guides the second continuous sheet so as to overlap the second continuous sheet onto the first continuous sheet so as to cover the first absorbent material supplied to the first continuous sheet, thereby forming the first composite sheet; and
a first reversing roll disposed adjacent to the first conveyance roll, around which the first composite sheet is wound so as to support the second continuous sheet of the first composite sheet, and which rotates in a direction opposite to that of the first conveyance roll to convey the first composite sheet:
and configured such that the first composite sheet is sucked onto a first support surface on which the first composite sheet is supported.

14. The apparatus for manufacturing an absorbent sheet according to claim 11, further comprising:
a second conveyor that is disposed between the second composite sheet forming apparatus and the press apparatus, has a second conveying surface that supports the second composite sheet and moves while sucking the second composite sheet, and conveys the second composite sheet toward the press apparatus.

15. The apparatus for manufacturing an absorbent sheet according to claim 11 or 14, wherein
the second composite sheet forming apparatus comprises:
a second conveyance roll around which the first composite sheet is wound and which conveys the second composite sheet;
a second absorbent material supplying apparatus that supplies the second absorbent material to the first composite sheet being wound around and conveyed by the second conveyance roll;
a second guide roll that guides the third continuous sheet so as to overlap the third continuous sheet onto the first composite sheet so as to cover the second absorbent material supplied to the second composite sheet, thereby forming the second composite sheet; and
a second reversing roll disposed adjacent to the second conveyance roll, around which the second composite sheet is wound so as to support the third continuous sheet of the second composite sheet, and which rotates in a direction opposite to that of the first conveyance roll to convey the second composite sheet;
and configured such that the second composite sheet is sucked onto a second support surface on which the second composite sheet is supported.

16. The apparatus for manufacturing an absorbent sheet according to claim 13, further comprising:
a first press roll that is disposed opposite the first reversing roll and presses the first composite sheet, which is wound around and conveyed by the first reversing roll, toward the first reversing roll.

17. The apparatus for manufacturing an absorbent sheet according to claim 15, further comprising:
a second press roll that is disposed opposite the second reversing roll and presses the second composite sheet, which is wound around and conveyed by the second reversing roll, toward the second reversing roll.

18. The apparatus for manufacturing an absorbent sheet according to claim 17, wherein the second belt and/or the second press roll and the second reversing roll are configured as the press apparatus that compresses the second composite sheet in the thickness direction.
